# EUROPEAN PATENT SPECIFICATION

(11) **EP 1 625 148 B1**
(45) Date of publication and mention of the grant of the patent: **26.09.2012**
(21) Application number: 04733537.7
(22) Date of filing: 18.05.2004
(51) Int. Cl.: C07K 14/16, C07K 7/06, C07K 14/47, C07K 14/62, C07K 14/705, C07K 14/435, A61K 48/00, C07K 7/08

(54) **PEPTIDE COMPLEX**
PEPTIDKOMPLEX
COMPLEXE PEPTIDIQUE

(30) Priority: 21.05.2003 US 472131 P; 21.05.2003 EP 03011498; 23.03.2004 EP 04006900
(43) Date of publication of application: 15.02.2006
(60) Divisional application: 10181570.2
(73) Proprietor: BIOTECH TOOLS S.A., 1120 Bruxelles (BE)
(72) Inventor: HENOT, Frederic, B-1040 Bruxelles (BE); LEGON, Thierry, B-3360 Korbeek Lo (BE); GALY-FAUROUX, Isabelle, 78670 Vilennes-sur-Seine (FR); DUCHATEAU, Jean, B-7060 Soignies (BE)
(74) Representative: von Kreisler Selting Werner
(86) International application number: PCT/EP2004/005316
(87) International publication number: WO 2004/104026

(56) References cited:
- WO-A-01/70772
- WO-A-01/79259
- WO-A-90/14835
- WO-A-97/06685
- WO-A-97/34002
- WO-A-02/099061
- US-A- 3 907 765
- US-A- 4 029 642
- US-B1- 6 312 711
- INOUYE K, ET AL.: "ENZYME-ASSISTED SEMISYNTHESIS OF HUMAN INSULIN" JOURNAL OF THE AMERICAN CHEMICAL SOCIETY, vol. 101, no. 3, 1979, pages 751-752, XP009088159
- AUGER I ET AL: "HLA-DR4 AND HLA-DR10 MOTIFS THAT CARRY SUSCEPTIBILITY TO RHEUMATOIDARTHRITIS BIND 70-KD HEAT SHOCK PROTINS" NATURE MEDICINE, NATURE PUBLISHING GROUP, NEW YORK, NY, US, vol. 2, no. 3, 1 March 1996 (1996-03-01), pages 306-310, XP000674788 ISSN: 1078-8956
- AUGER I ET AL: "HLA-DR4 AND HLA-DR10 MOTIFS THAT CARRY SUSCEPTIBILITY TO RHEUMATOIDARTHRITIS BIND 70-KD HEAT SHOCK PROTINS" NATURE MEDICINE, NATURE PUBLISHING, CO, US, vol. 2, no. 3, 1 March 1996 (1996-03-01), pages 306-310, XP000674788 ISSN: 1078-8956
- CWIKLINSKA H ET AL: "Heat shock protein 70 associations with myelin basic protein and proteolipid protein in multiple sclerosis brains" INTERNATIONAL IMMUNOLOGY, OXFORD UNIVERSITY PRESS, GB, vol. 15, no. 2, 1 February 2003 (2003-02-01), pages 241-249, XP009018025 ISSN: 0953-8178
- BECKER THALIA ET AL: "CD40, an extracellular receptor for binding and uptake of Hsp70-peptide complexes" THE JOURNAL OF CELL BIOLOGY, ROCKEFELLER UNIVERSITY PRESS, US, vol. 158, no. 7, 30 September 2002 (2002-09-30), pages 1277-1285, XP002245261 ISSN: 0021-9525

## Description

The present invention relates to complexes of heat shock proteins and tolerogenic peptides.

It is known that complexes of peptides together with heat shock proteins are suitable for inducing tolerance.

US 6,312,711 discloses a pharmaceutical or food composition intended for treating pathologies associated with graft rejection or an allergic or autoimmune reaction comprising the administration of a complex of a stress protein and epitopes of an antigenic structure.

K. Inouye et al. in Journal of the American Chemical Society 101 (1979), 751 - 752 describe enzyme assisted semi-synthesis of human insulin.

US 3,907,765 describes a process for preparing octapeptide intermediates for human insulin and intermediates.

US 4,029,642 describes a process for the manufacture of human insulin by condensation of a B₂₃₋₃₀ octapeptide.

I. Auger et al. in Nature Medicine 2 (1996) 306 - 310 describe binding of peptides to DnaK and the role in rheumatoid arthritis.

Surprisingly, the inventors of the present invention have been able to isolate small peptides which in complex with heat shock proteins are especially useful for prevention or treatment of allergy, graft rejection or autoimmune diseases.

The inventors identified the following sequences

| | |
|---|---|
| GFFYTPK (insulin 23-29) | SEQ ID No 1 |
| GFFYTPKT (insulin 23-30) | SEQ ID No 2. |

Preferably these sequences are prepared by hydrolysis of natural occurring peptides or proteins.

In one aspect of the invention, the invention provides a complex comprising at least one heat shock protein (HSP) and at least one peptide selected from Seq. ID Nos. 1 to 2.

In a preferred embodiment, the HSP is a bacterial HSP and more preferably a bacterial HSP from a saprophytic bacteria. Suitable HSP can be selected from hsp40, hsp70, grpE, hsp90, CPN60 (hsp60), FK506-binding proteins, gp96, ca-Irecticulin, hsp110, grp170 and hsp100, alone or in combination.

According to the invention either complete HSP or fragments of the HSP can be used. A preferred fragment is the polypeptide binding domain of the heat shock protein.

In a further aspect, the invention provides a nucleic acid encoding at least one HSP and at least one peptide selected from Seq. ID No. 1 to Seq. ID No. 2. This nucleic acid can be used to express the corresponding peptide in vitro or in a cell or in a patient. For expression in a patient, a gene delivery vehicle would be necessary. Therefore, the gene delivery vehicle comprising at least the nucleic acid of the invention is also part of the invention.

Such a gene delivery vehicle would transfect a cell. Therefore, an isolated cell transfected by at least one gene delivery vehicle of the invention is also part of the invention.

Either the complex or the gene delivery vehicle could be administered to patient in need thereof. Therefore, a pharmaceutical or food composition comprising the complex or the gene delivery vehicle of the present invention is also an aspect of the invention.

The complex of the present invention can be prepared by hydrolyzing *in-vitro* at least one protein with at least one protease to obtain hydrolyzed fragments and combining *in-vitro* the hydrolyzed fragments with at least one heat shock protein to obtain at least one complex.

Alternatively, the complex of the present invention can be prepared by synthesizing the peptides identified as Seq. ID No. 1 to Seq. ID No. 2 and combining the synthesized peptide with at least one heat shock protein. Suitable procedures are described by Merrifield, 1963, J. Am. Chem. Soc. 85:2149.

In a preferred embodiment, the peptide-HSP complex is formed under reducing conditions in a buffer containing at least one reducing agent. Suitable reducing agents are for example dithiothreitol and beta-mercaptoethanol. The most preferred concentrations of the reducing agent are from 0.001 mM to 700 mM, and more preferably between 0.1 mM and 50 mM.

In another embodiment, the peptide-HSP complex is formed under oxidative conditions in a buffer containing at least one oxidative agent. A suitable oxidative agent is for example hydrogen peroxide. The most preferred concentrations of the oxidative agent are from 0.1 mM to 100 mM, and more preferably between 0.5 mM and 20mM.

In another preferred embodiment, the peptide-HSP complex is formed in a buffer which contains neither a reducing agent nor an oxidative agent.

A further aspect of the invention is to use the complex of the present invention as a carrier for the delivery of biologically active molecules. The peptides can be linked to biologically active molecules e.g. nucleic acids, peptides, polypeptides, proteins, polysaccharides, lipids, drugs, and can then be combined with the heat shock protein.

The complex of the present invention or the gene delivery vehicle of the invention are useful for the prevention or treatment of allergy, graft rejection or autoimmune diseases.

The complex of the present invention can also be used for the induction of an *in-vitro* cellular reponse comprising cells able to secrete immunosuppressive cytokines after exposure to at least one antigen comprising at least one sequence selected from SEQ ID No 1 to SEQ ID No 2.

The complex of the invention is e.g. useful as a diagnostic composition or in a method for the in vitro diagnosis of a disease comprising the steps of
- bringing a biological fluid of an animal containing proteins into contact with the diagnostic composition of the invention
- measuring binding of said proteins with said diagnostic composition
wherein binding indicates a disease of the animal.

Another use of the complex of the invention is to provide with antibodies specific for at least one complex of the present invention. The antibody can be a polyclonal antibody, a monoclonal antibody or fragments of such as but not limited to Fab and F(ab')₂.

The antibody can be used for the prevention or treatment of allergy, graft rejection or autoimmune disease. Also the antibody can be used in a method for the in vitro diagnosis of a disease such as allergy, graft rejection or autoimmune disease.

As an alternative to direct administration of the heat shock protein peptide complex, one or more polynucleotide constructs may be administered which encode heat shock protein and target peptide in expressible form. The expressible polynucleotide constructs are introduced into cells using in vivo or in vitro methods.

Amino-acid sequences and nucleotides sequences of HSP and target proteins are generally available in sequences databases such as GenBank and Swiss-Prot.

DNA sequences can be amplified from genomic or cDNA by PCR using specific primers designed from the known sequences. "PCR" refers to the technique of polymerase chain reaction as described in Saikl, et al., Nature 324:163 (1986); and Scharf et al., Science (1986) 233:1076-1078; and US patent No. 4,683,195; and US patent No. 4,683,202. A nucleotide sequence encoding a polypeptide of any desired length can be generated by PCR. PCR can be carried out e.g. by use of a thermal cycler and a thermo-resistant DNA polymerase.

Methods of incorporating particular nucleic acids into expression vectors are well known to those of skill in the art, but are described in detail in, for example, Sambrook et al., Molecular Cloning: A Laboratory Manual (2nd ed.), Vols. 1 to 3, Cold Spring Harbor Laboratory, (1989) or Current Protocols in Molecular Biology, F. Asubel et al., ed. Green Publishing and Wiley-Interscience, New York (1987). To construct an expressible polynucleotide, a region encoding the heat shock protein and peptide is prepared as discussed above and inserted into a mammalian expression vector operatively linked to a suitable promoter such as the SV40 promoter, the cytomegalovirus (CMV) promoter or the Rous sarcoma virus (RSV) promoter. The resulting construct may then be used as a vaccine for genetic immunization. The nucleic acid polymer(s) could also be cloned into a viral vector. Suitable vectors include but are not limited to retroviral vectors, adenovirus vectors, vaccinia virus vectors, pox virus vectors and adenovirus-associated vectors.

Alternatively, a polynucleotide containing a sequence encoding the HSP and a gene encoding a desired peptide may be introduced respectively into two different vectors each capable of coexistence and replication in the same host.

The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, complexation of the polynucleotide(s) in liposomes, and direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e.g., Hep G2), and a number of other cell lines.

### Examples

### Protein preparation

Before enzymatic digestions, the proteins of interest are extensively washed with water by centrifugation through a centricon YM-03 or YM-10 assembly to remove any low molecular weight material loosely associated with it.

### Trypsin digestion

One to five milligrams of the protein of interest is dissolved in 1 to 5 mL of phosphate buffer 40 mM pH 8.0 (final concentration of 1 mg/mL). The solution is incubated for 10 minutes at 100°C. The solution is then rapidly cooled at 4°C, and 18 to 90 µL of β-mercaptoethanol is added (1.8% v/v). The resulting solution is incubated at 37°C for 10 minutes and 20 to 100 µL of trypsin solution (10 mg/mL of Tris●HCl 40 mM pH 8.0; final ratio (w/w) protease/protein of 1:100) is added to the protein solution.

The resulting solution is incubated at 37°C for six hours. The solution is then centrifuged through a centricon YM-10 assembly to remove the remaining protein and trypsin.

### DnaK.ATP complex preparation

25 µL of ATP solution (4.5 mg/mL of buffer 1 (25 mM HEPES, 10 mM KCI, 3 mM magnesium chloride, 5 mM beta-mercaptoethanol, pH 7.5) is added to 400 µL of DnaK (2 mg/mL of buffer 1). The solution is incubated at 20°C for one hour, and then centrifuged through a centricon YM-10 assembly to remove any low molecular weight material loosely associated with DnaK. The large molecular weight fraction is removed, and washed extensively with buffer 1 by ultrafiltration using a centricon YM-10.

### In vitro production of stress protein-peptide complexes. Isolation of the bound peptides

The ultra filtrated trypsin digestion is mixed with the ATP-pre-treated DnaK to give at least a 1:1 (w:w) DnaK:peptides ratio. Then, ADP is added (1 mM final) and the mixture is incubated for at least one hour at 25°C in the suitable buffer 1. The preparations are centrifuged through a centricon YM-10 assembly to remove the remaining unbound peptides. The low and the large molecular weight fractions are recovered. The large molecular weight fraction containing DnaK-peptide complexes is washed extensively with buffer 1 containing 1 mM ADP by ultrafiltration using a centricon YM-10. The bound peptides are eluted by incubating the HSP-peptide complexes in a low pH buffer. A last ultrafiltration using a centricon YM-10 is removing the large molecular weight fraction.

### HPLC analysis

The resulting low molecular weight fractions are fractionated by reverse phase high pressure liquid chromatography (HPLC) using a Vydac C18 reverse phase column (HP32, 201TP52 C18, 250/2.1 mm, 5 µm (available from Vydac)). The elution of the peptides can be monitored at both OD 214 nm and OD 280 nm.

### Biological studies

It is a model wherein NOD (Not Obese Diabetic) mice are treated after the onset of the auto-immune disease.

After the onset of the first signs of diabetes, 500 normal Langerhans islets from young NOD mice are grafted under the renal capsule of diabetic animals. Glycosuria and glycemia are then monitored daily. Mice are considered diabetic when a glucosuria is detected and glycemia exceeds 12 mmole/L (2,16 g/L) during two consecutive days. The first day of hyperglycemia is considered as the start of relapse.

### Treatment of mice

All treatments were started on the first day after the onset of the disease, that is the day before transplantation. Mice were treated by sublingual injections, one dose each two days, to achieve the total doses:
Group 1: Peptides (1 µg) + HSP (1 µg)
Group 2: Peptides (1 µg)
Group 3: HSP (1 µg)
Group 4: buffer

### Clinical outcomes

In non-treated NOD mice, the average delay before a relapse occurs is about 11 to 12 days. Considering that a delay exceeding 14 days results from a therapeutic effect, one notices that, in the group treated with complexes, the proportion of delays exceeding 14 days is 4/7 (57%). In the other group treated with either peptides, buffer or HSP alone, the proportion is 2/6 (33%). Thus, there is a therapeutic effect of the combination between an HSP and peptides given orally.

The mRNAs of interferon-γ and IL-4 have been assayed by PCR amplification in various tissues: the graft, the spleen and, as a control, the kidney. The results are expressed as ratios of the amount of interferon-γ mRNA vs the amount of actine mRNA. IL-4 was not detected in any sample, whereas interferon-γ was found in the spleen at levels that were rather similar in all the groups.

In contrast, some interesting differences were observed in the grafted islets. The level of interferon-y was significantly lower in the group treated with peptides-HSP complexes. It is thus possible that the complexes had induced in the Peyer's patches regulatory T cells that, after their migration into the graft, have inhibited the T_{H}1 cells that contribute to the destruction of the grafted islets by secreting cytokines such as interferon-γ.

### Figures

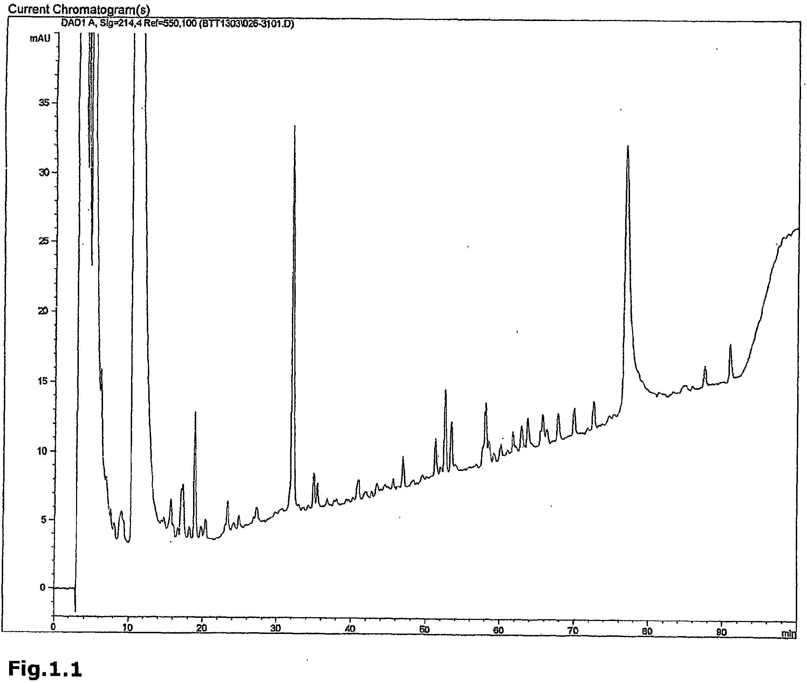

Figure 1.1: peptides (MW < or = 10 kDa) generated by trypsin-cleavage of Derp1 (not according to the invention).

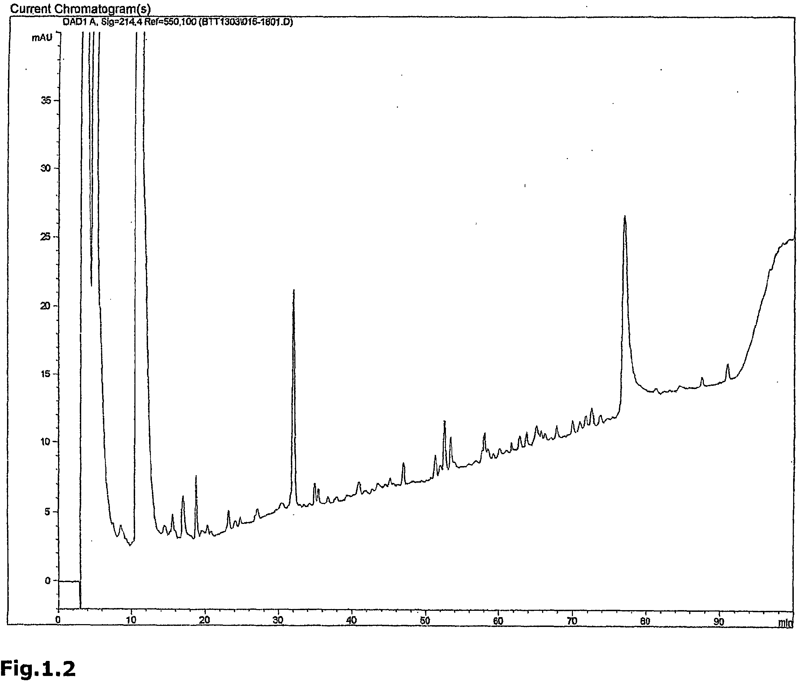

Figure 1.2: unbond peptides (MW < or 10 kDa) to DnaK, from the trypsin-cleavage of Derp1. (not according to the invention)

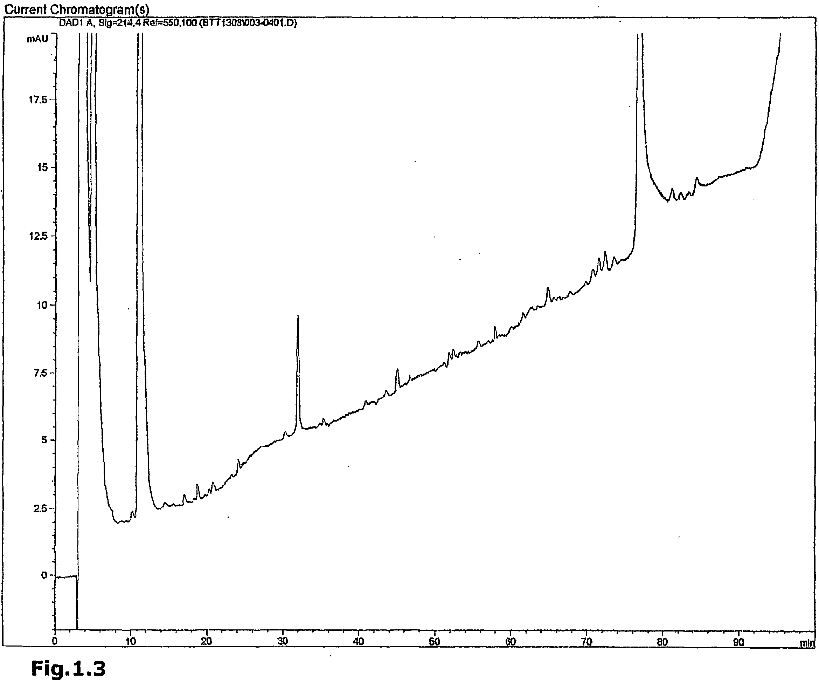

Figure 1.3: peptides from the trypsin-cleavage of Derp1 (MW < or = 10 kDa) that were bound to DnaK (not according to the invention).

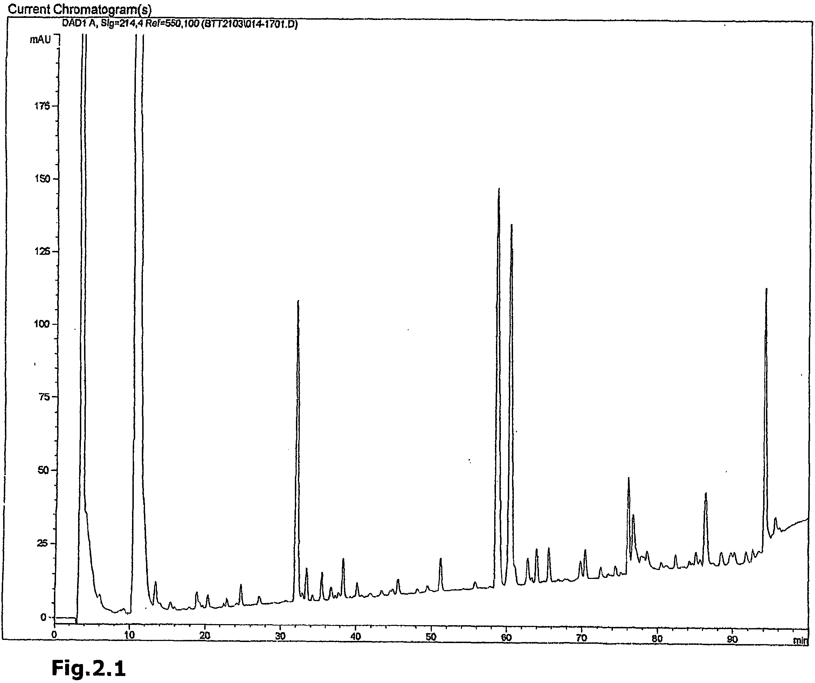

Figure 2.1: peptides (MW < or 10 kDa) generated by trypsin-cleavage of insulin.

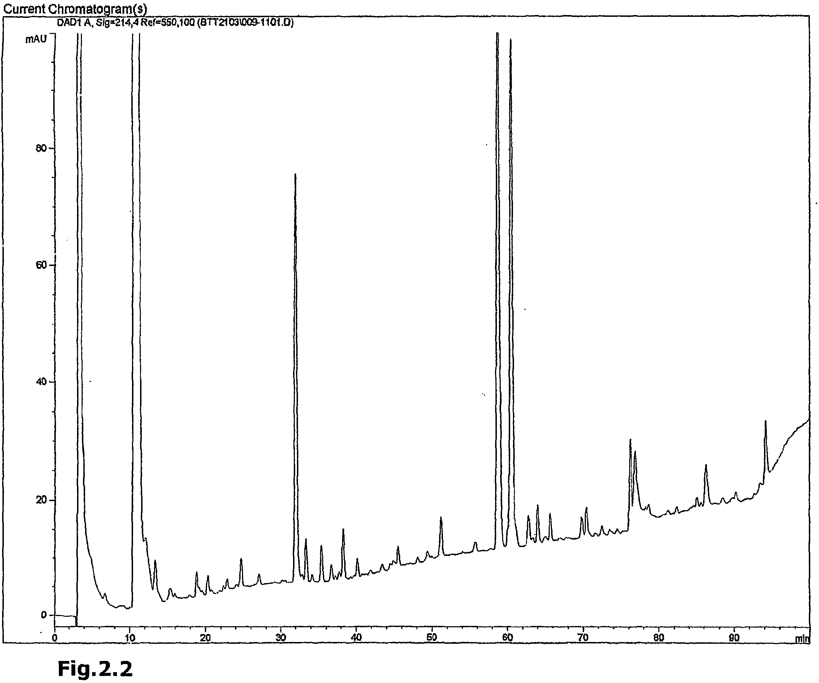

Figure 2.2: unbound peptides (MW < or 10 kDa) to DnaK, from the trypsin-cleavage of insulin.

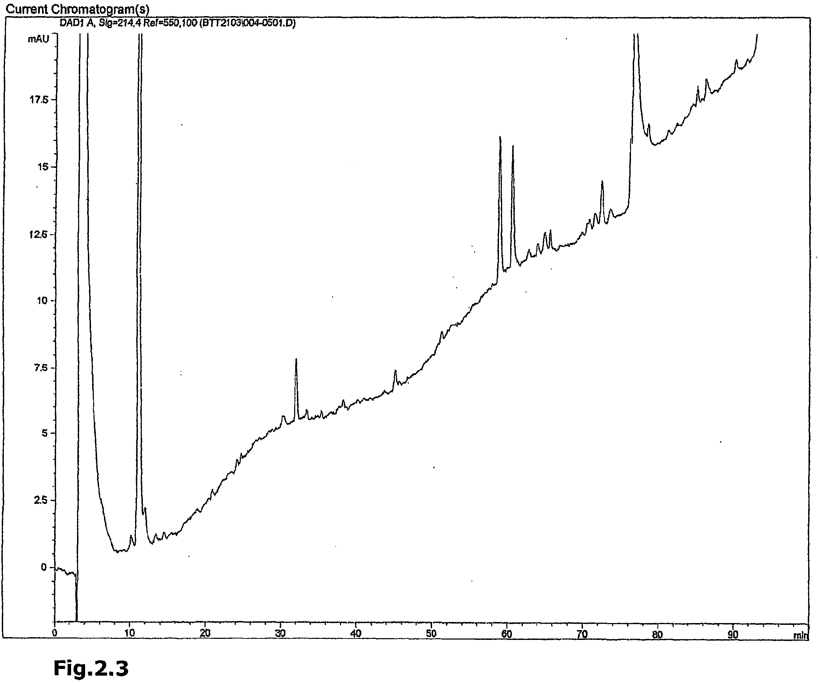

Figure 2.3: peptides from the trypsin-cleavage of insulin (MW < or = 10 kDa) that were bound to DnaK.

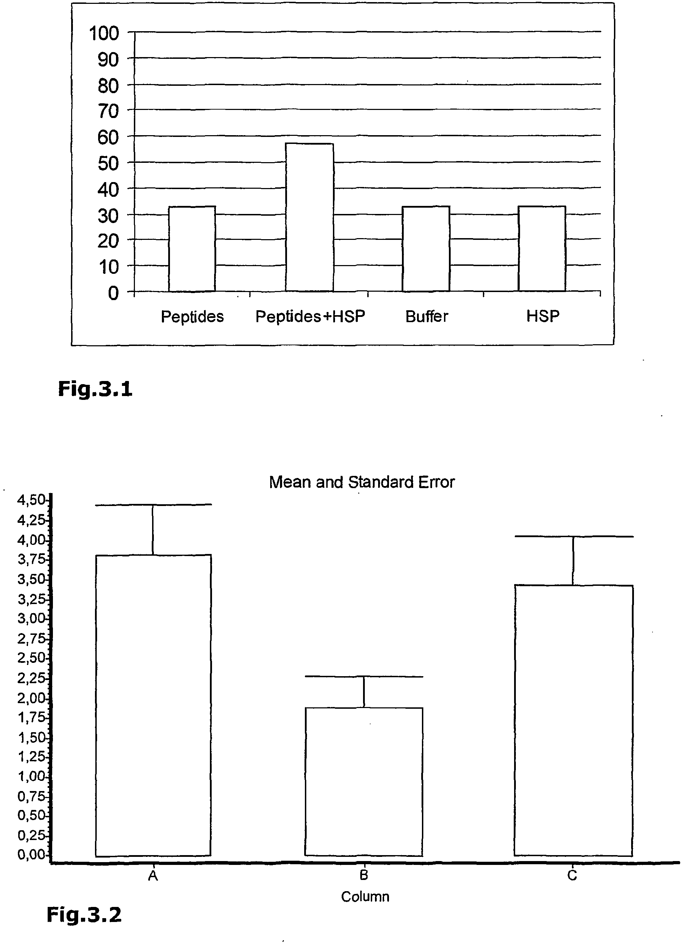

Figure 3.1: proportion of delays before relapse exceeding 14 days.
Figure 3.2: expression of IFN-γ mRNA in the grafted islets. A: group 2 (peptides); B: group 1 (HSP + peptides); C: buffer.

## Claims

1. A complex comprising at least one heat shock protein (HSP) and at least one peptide
| | |
|---|---|
| GFFYTPK (insulin 23-29) | SEQ ID No 1 |
| GFFYTPKT (insulin 23-30) | SEQ ID No 2. |

2. The complex according to claim 1, wherein the HSP is a bacterial HSP.

3. The complex according to of claim 1 or 2, wherein the HSP is selected from hsp40, hsp70, grpE, hsp90, CPN60 (hsp60), FK506-binding proteins, gp96, calrecticulin, hsp110, grp170 and hsp100.

4. The complex according to any of claims 1 to 3, wherein the heat shock protein is the polypeptide binding domain of a heat shock protein.

5. A nucleic acid encoding at least one HSP and at least one peptide selected from SEQ ID No 1 or 2.

6. A gene delivery vehicle comprising at least one nucleic acid according to claim 5.

7. An isolated cell transfected by at least one gene delivery vehicle according to claim 6.

8. A pharmaceutical or food compositions comprising the complex of any of claims 1 to 4 or a gene delivery vehicle of claim 6.

9. A method for preparing a complex according to any one of claims 1 to 4 comprising the steps of
• hydrolyzing *in-vitro* at least one protein with a protease to obtain hydrolyzed fragments, having Seq. ID No. 1 or 2
• combining *in-vitro* the hydrolyzed fragments with at least one heat shock protein to obtain a complex.

10. The method of claim 9, wherein unbound hydrolyzed fragments are separated from the complex.

11. The complex of any of claims 1 to 4 or the gene delivery vehicle of claim 6 for use in the prevention or treatment of allergy, graft rejection or autoimmune disease.

12. Use of the complex of any of claims 1 to 4 for the *in-vitro* induction of a cellular response comprising cells secreting immunosuppressive cytokines after exposure to at least one antigen comprising at least SEQ ID No 1 or 2

## Patentansprüche

1. Komplex, der wenigstens ein Hitzeschockprotein (HSP) und wenigstens ein Peptid
| | |
|---|---|
| GFFYTPK (Insulin 23-29) | SEQ ID Nr. 1 |
| GFFYTPKT (Insulin 23-30) | SEQ ID Nr. 2 |
umfasst.

2. Komplex gemäß Anspruch 1, wobei das HSP ein bakterielles HSP ist.

3. Komplex gemäß Anspruch 1 oder 2, wobei das HSP aus hsp40, hsp70, grpE, hsp90, CPN60 (hsp60), FK506-bindenden Proteinen, gp96, Calrecticulin, hsp110, grp170 und hsp100 ausgewählt ist.

4. Komplex gemäß einem der Ansprüche 1 bis 3, wobei das Hitzeschockprotein die Polypeptid-bindende Domäne eines Hitzeschockproteins ist.

5. Nucleinsäure, die wenigstens ein HSP und wenigstens ein Peptid aus SEQ ID Nr. 1 oder 2 codiert.

6. Gentransfervehikel, das wenigstens eine Nucleinsäure gemäß Anspruch 5 umfasst.

7. Isolierte Zelle, die mit wenigstens einem Gentransfervehikel gemäß Anspruch 6 transfiziert ist.

8. Pharmazeutische oder Nahrungsmittelzusammensetzungen, die den Komplex gemäß einem der Ansprüche 1 bis 4 oder ein Gentransfervehikel gemäß Anspruch 6 umfassen.

9. Verfahren zur Herstellung eines Komplexes gemäß einem der Ansprüche 1 bis 4, umfassend die Schritte:
• Hydrolysieren wenigstens eines Proteins in vitro mit einer Protease, wobei man Hydrolysefragmente erhält, die SEQ ID Nr. 1 oder 2 aufweisen;
• Kombinieren der Hydrolysefragmente in vitro mit wenigstens einem Hitzeschockprotein, wobei man einen Komplex erhält.

10. Verfahren gemäß Anspruch 9, wobei ungebundene Hydrolysefragmente von dem Komplex abgetrennt werden.

11. Komplex gemäß einem der Ansprüche 1 bis 4 oder Gentransfervehikel gemäß Anspruch 6 zur Verwendung bei der Prävention oder Behandlung von Allergien, Transplantatsabstoßung oder Autoimmunkrankheiten.

12. Verwendung des Komplexes gemäß einem der Ansprüche 1 bis 4 zur in-vitro-Induktion einer zellulären Antwort, die Zellen umfasst, die nach Einwirkung wenigstens eines Antigens, das wenigstens SEQ ID Nr. 1 oder 2 umfasst, immunsuppressive Cytokine sezernieren.

## Revendications

1. Complexe comprenant au moins une protéine de choc thermique (PCT) et au moins un peptide
| | |
|---|---|
| GFFYTPK (insuline 23-29) | SEQ ID N° : 1 |
| GFFYTPKT (insuline 23-30) | SEQ ID N° : 2. |

2. Complexe selon la revendication 1, dans lequel la PCT est une PCT bactérienne.

3. Complexe selon la revendication 1 ou 2, dans lequel la PCT est choisie parmi pct 40, pct 70, grpE, pct90, CPN60 (hsp60), les protéines de liaison à FK506, gp96, la calrecticuline, pct 110, grp170 et pct 100.

4. Complexe selon l'une quelconque des revendications 1 à 3, dans lequel la protéine de choc thermique est le domaine de liaison au polypeptide d'une protéine de choc thermique.

5. Acide nucléique codant pour une PCT et au moins un peptide choisi parmi SEQ ID NO : 1 ou 2.

6. Véhicule de livraison génique comprenant au moins un acide nucléique selon la revendication 5.

7. Cellule isolée transfectée par au moins un véhicule de livraison génique selon la revendication 6.

8. Compositions pharmaceutiques ou alimentaires comprenant le complexe de l'une quelconque des revendications 1 à 4 ou un véhicule de livraison génique de la revendication 6.

9. Procédé de préparation d'un complexe selon l'une quelconque des revendications 1 à 4, comprenant les étapes consistant à
• hydrolyser *in vitro* au moins une protéine avec une protéase pour obtenir des fragments hydrolysés, ayant SEQ ID NO : 1 ou 2
• combiner *in vitro* les fragments hydrolysés avec au moins une protéine de choc thermique pour obtenir un complexe.

10. Procédé selon la revendication 9, dans lequel les fragments hydrolysés non liés sont séparés du complexe.

11. Complexe selon l'une quelconque des revendications 1 à 4 ou véhicule de livraison génique selon la revendication 6, à utiliser dans la prévention ou le traitement d'une allergie, d'un rejet de greffe ou d'une maladie auto-immune.

12. Utilisation du complexe de l'une quelconque des revendications 1 à 4, pour l'induction *in vitro* d'une réponse cellulaire comprenant des cellules sécrétant des cytokines immunosuppressives après exposition à au moins un antigène comprenant au moins SEQ ID NO : 1 ou 2.
